# EUROPEAN PATENT APPLICATION

(11) **EP 4 464 347 A1**
(43) Date of publication of application: **20.11.2024**
(21) Application number: 22920427.6
(22) Date of filing: 31.10.2022
(51) Int. Cl.: A61M 5/158, A61M 5/34, A61M 5/46

(54) **NEEDLE ASSEMBLY AND ADMINISTRATION DEVICE**

(30) Priority: 14.01.2022 JP 2022004167
(71) Applicant: TERUMO Kabushiki Kaisha, Tokyo 151-0072 (JP)
(72) Inventor: TANAKA, Ryo, Ashigarakami-gun, Kanagawa 259-0151 (JP); ITO, Eriko, Ashigarakami-gun, Kanagawa 259-0151 (JP); IWASE, Yoichiro, Ashigarakami-gun, Kanagawa 259-0151 (JP); AKAHORI, Yuta, Ashigarakami-gun, Kanagawa 259-0151 (JP)
(74) Representative: Casalonga
(86) International application number: PCT/JP2022/040619
(87) International publication number: WO 2023/135905

(57) **Abstract**

A needle assembly includes an injection needle that includes a needle tube in which a blade surface is formed at a needle tip, and a needle hub that holds the injection needle. The needle hub includes a holding portion that has a penetration region through which the injection needle penetrates and that holds the injection needle penetrating the penetration region, and an insertion portion that is disposed on the distal end side of the holding portion and that guides a proximal end portion of the injection needle to the penetration region such that the puncture direction of the injection needle and the center line of the penetration region overlap with each other.

## Description

### Technical Field

The present invention relates to a needle assembly and an administration device.

### Background

Conventionally, as a method for administering a drug such as a vaccine to a human, so-called "intradermal administration" has been known, in which a skin upper layer portion where a large number of immunocompetent cells are present is used as a target site. It has been studied to reduce the dose of a drug by performing intradermal administration. In a case where the administration target is a human, the intradermal administration described above is generally intended for administration to a dermis layer forming a "skin upper layer portion" among skin formed by an "epidermis layer" having a thickness of 50 um to 200 um from the skin surface, a "dermis layer" having a thickness of 0.5 to 3.5 mm continuing from the epidermis layer, and a "subcutaneous tissue layer" deeper than the dermis layer. The "skin upper layer portion" refers to the epidermis layer and the dermis layer of the skin.

Patent literature 1 below discloses a device capable of intradermal administration. In the device of Patent literature 1, a needle assembly inserts the proximal end side of a needle tube of an injection needle into a mounting hole of an elastic member and liquid-tightly holds the needle tube at the close contact portion. Then, in the device of Patent literature 1, the distal end of a syringe in which the drug can be accommodated is pressed against an end surface of the elastic member, and the syringe and the needle assembly are fitted with the elastic member and the injection needle brought into close contact with each other by deformation of the elastic member and exerting a pressure resistance. Thus, leakage of the drug to the needle tip side can be prevented.

### Citation List

### Patent Literature

Patent Literature 1: WO2016/052169 A

### SUMMARY OF INVENTION

### Technical Problem

Incidentally, the thickness of a biological tissue such as a human mucous membrane or the skin of a small non-human animal which is often used in animal experiments and the like is thinner than the thickness of the upper layer portion of the human skin (epidermis layer and dermis layer). Therefore, in order to administer a drug to these administration sites, an injection needle (e.g., 36G injection needle) having an outer diameter smaller than that of a generally used injection needle is sometimes used.

When the use of the device described in WO 2016/052169 A is considered as an administration device, this device has a configuration in which an injection needle having an application size of 26G to 33G is inserted into the mounting hole of the close contact portion. Therefore, in order to prevent liquid leakage using an injection needle having an inapplicable outer diameter size, it is necessary to form the diameter of the mounting hole smaller than the design and to hold the injection needle in a liquid-tight manner with respect to the close contact portion.

However, there is a limit to adjusting the diameter of the mounting hole of the device of WO 2016/052169 A, and it is not realistic to reduce the diameter of the mounting hole from the viewpoint of mass production efficiency and the like.

In addition, when an injection needle having a small outer diameter is applied to the device of WO 2016/052169 A, it is also conceivable to bring the injection needle and the mounting hole into close contact with each other by deformation of the elastic member. However, in a state where a gap is generated between the diameter of the mounting hole and the outer diameter of the injection needle, sufficient pressure resistance due to deformation of the elastic member cannot be obtained, and there is a high possibility that liquid leaks from the gap between the injection needle and the mounting hole.

As a result of intensive studies on the specific problem for achieving drug administration to a biological tissue thinner than the epidermis layer and the dermis layer of human as described above, the present inventors have devised a needle assembly and an administration device capable of solving the problem.

At least one embodiment of the present invention has been made in view of the above circumstances, and specifically, an object thereof is to provide a needle assembly and an administration device capable of holding an injection needle in a liquid-tight manner regardless of the outer diameter of the injection needle.

### Solution to Problem

A needle assembly according to one aspect of the present invention includes an injection needle that includes a needle tube in which a blade surface is formed at a needle tip, and a needle hub that holds the injection needle. The needle hub includes a holding portion that has a penetration region through which the injection needle penetrates and that holds the injection needle penetrating the penetration region, and an insertion portion that is disposed on a distal end side of the holding portion and that guides a proximal end portion of the injection needle to the penetration region such that a puncture direction of the injection needle and a center line of the penetration region overlap with each other.

In addition, an administration device according to the present invention includes the needle assembly, and a syringe that has a first tubular portion disposed on the proximal end side of the needle hub and a second tubular portion disposed on the proximal end side of the first tubular portion and having an outer diameter larger than an outer diameter of the first tubular portion, and that is connected to the needle hub of the needle assembly. Advantageous Effects of Invention

According to the needle assembly and the administration device according to the present invention, it is possible to hold the injection needle in a liquid-tight manner regardless of the outer diameter of the injection needle. Therefore, even an injection needle having a small outer diameter of, for example, 36G or less, which is used when intradermal administration to a site thinner than an upper layer portion of the human skin, can be held in a liquid-tight manner and prevent liquid leakage.

### BRIEF DESCRIPTION OF DRAWINGS

FIG. 1 is a perspective view of an administration device according to the present embodiment;
FIG. 2 is an exploded view of a needle assembly according to the present embodiment;
FIG. 3 is a partial cross-sectional view of a needle hub and a syringe according to the present embodiment;
FIG. 4 is a partial cross-sectional view of the needle hub according to the present embodiment;
FIG. 5 is an enlarged cross-sectional view of a Z region in FIG. 4;
FIG. 6 is a partial cross-sectional view of a state in which an injection needle is attached to the needle hub according to the present embodiment;
FIG. 7A is a schematic cross-sectional view illustrating a form of a proximal end portion of the injection needle according to the present embodiment;
FIG. 7B is a schematic perspective view illustrating another form of the proximal end portion of the injection needle according to the present embodiment;
FIG. 8A is a schematic cross-sectional view illustrating an operation when the injection needle is attached to the needle hub according to the present embodiment;
FIG. 8B is a schematic cross-sectional view illustrating an operation when attaching the injection needle to the needle hub according to the present embodiment;
FIG. 8C is a schematic cross-sectional view illustrating an operation when attaching the injection needle to the needle hub according to the present embodiment;
FIG. 9 is a graph showing results of a pressure resistance test of Example 1 and Comparative Example 1;
FIG. 10 is a graph showing results of a pressure resistance test of Example 2 and Comparative Example 2; and
FIG. 11 is a graph showing results of a pressure resistance test of Example 3 and Comparative Example 3.

### DETAILED DESCRIPTION

Hereinafter, a mode for carrying out the present invention will be described in detail with reference to the drawings. Embodiments herein are illustrated to embody the technical idea of the present invention and do not limit the present invention. Furthermore, other conceivable modes, examples, operation techniques, and the like, that can be implemented by those skilled in the art without departing from the gist of the present invention are all included in the scope and gist of the present invention and included in the invention described in the claims and the scope of equivalents thereof.

Moreover, for convenience of illustration and ease of understanding, the drawings attached to the present specification may be schematically represented by changing a scale, an aspect ratio, a shape, and the like from actual ones as appropriate, but are merely examples, and do not limit the interpretation of the present invention.

Note that, in the following description, ordinal numerals such as "first" and "second" will be given, but are used for convenience and do not define any order unless otherwise specified.

### [Configuration]

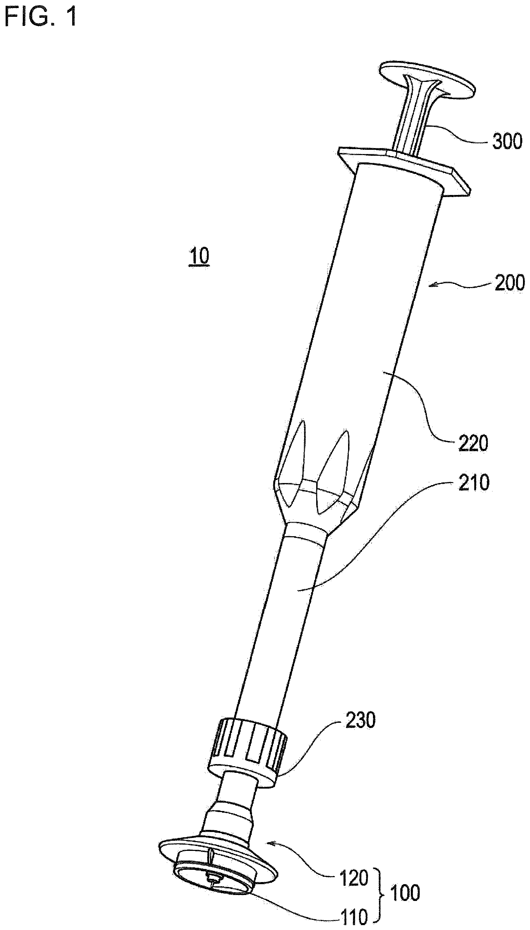

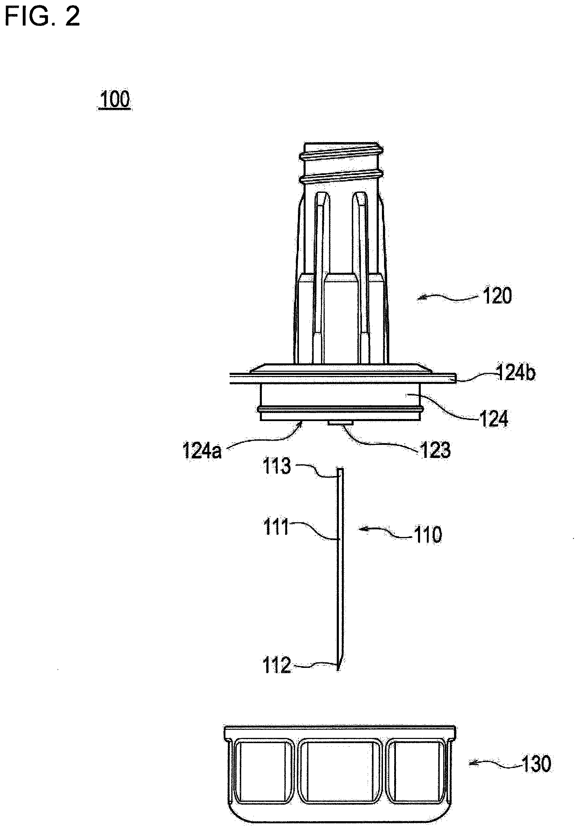

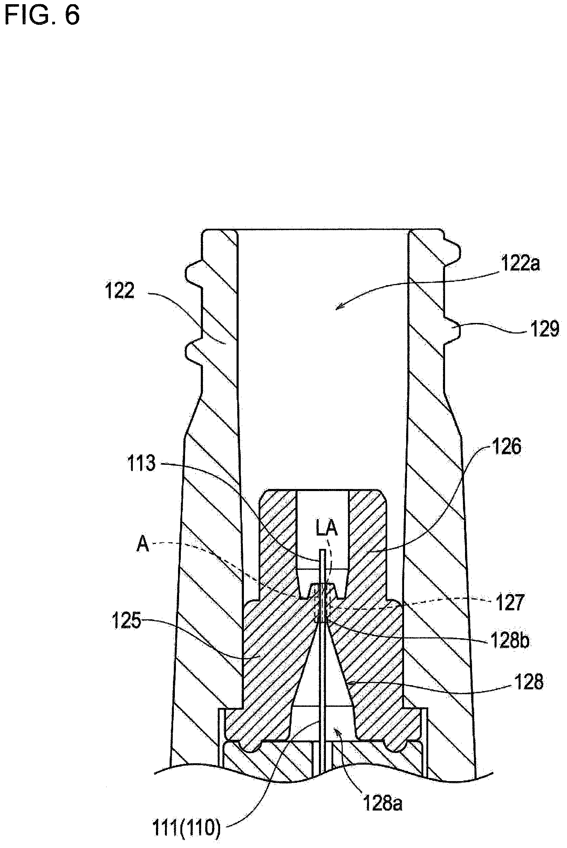

An administration device 10 according to the present embodiment will be described with reference to FIGS. 1 to 7B as appropriate. FIG. 1 schematically illustrates the overall configuration of the administration device 10.

The administration device 10 can be suitably used when a drug is administered to a biological tissue (mucous membrane of human, dermis layer of small non-human animal, and the like) thinner than the upper layer portion of the human skin (epidermis layer and dermis layer) to be the administration site in a human or a small non-human animal to be administered. In addition, the form of administration to these administration sites is referred to as "intradermal administration".

The small non-human animal (in particular, small non-human laboratory animal) to be administered is preferably a warm-blooded animal. Examples of the warm-blooded animal to be used include mammals (e.g., rat, mouse, musk shrew, gerbil, rabbit, mouse hare, guinea pig, and hamster) and birds (e.g., chicken, pigeon, quail, and the like). Among them, mammals are more preferable, rodents (rat, mouse, rabbit, guinea pig, hamster, and the like) are still more preferable, rat, mouse, and rabbit are particularly preferable, and rat is most preferable. Genetically modified animals such as transgenic animals and disease model animals may be used as small non-human animals.

First, the administration device 10 according to the present embodiment will be described. As illustrated in FIG. 1, the administration device 10 includes a needle assembly 100 including a needle hub 120 holding an injection needle 110, and a syringe 200 connectable to the needle assembly 100.

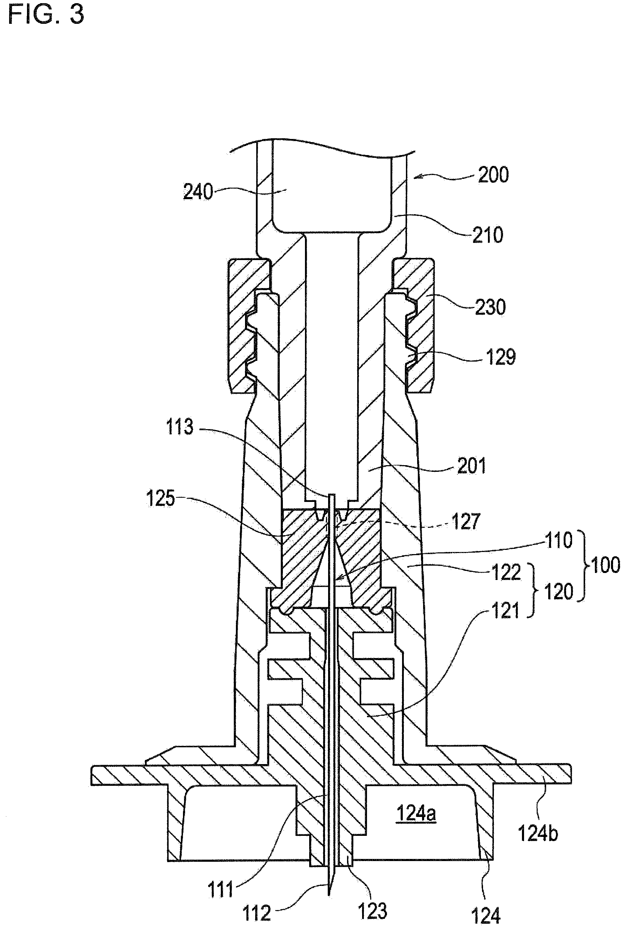

As illustrated in FIG. 3, a liquid chamber 240 capable of accommodating a drug (liquid medicine) is provided inside the syringe 200. In the liquid chamber 240 of the syringe 200, a plunger 300 for feeding the drug from the liquid chamber 240 into the lumen of a needle tube 111 of the injection needle 110 is inserted so as to be movable forward and backward.

In the present embodiment, the extending direction (longitudinal direction) of the injection needle 110 and the syringe 200 is also referred to as an "axial direction". Further, a side of a needle tip 112 of the injection needle 110 is referred to as a "distal end side", and an end side opposite to the distal end side is referred to as a "proximal end side". That is, for example, in FIG. 3, the lower side in the drawing is the "distal end side", and the upper side in the drawing is the "proximal end side".

A medical worker (hereinafter referred to as "user") including an operator or the like can intradermally administer a drug to an administration target through a distal end opening formed in the needle tip 112 by moving the plunger 300 toward the distal end side of the syringe 200 in a state where the needle tip 112 of the injection needle 110 is disposed at an administration site (e.g., in dermis layer of skin of small non-human animal).

The administration device 10 can be configured as, for example, a disposable device in which the liquid chamber 240 is filled with a drug at the time of intradermal administration, and the device is disposed of every time the filled drug is administered. Note that the administration device 10 can also be configured as a prefilled device in which the liquid chamber 240 is filled with a drug in advance before intradermal administration. In addition, while the type of drug (including liquid medicine to be intradermally administered) to be accommodated in the liquid chamber 240 is not particularly limited, the drug is at least dischargeable from the injection needle 110.

### <Syringe>

As illustrated in FIG. 1, the syringe 200 includes a first tubular portion 210 provided with a drug discharge portion 201 at the distal end, a second tubular portion 220 disposed on the proximal end side of the first tubular portion 210, and a lock mechanism 230 disposed on the distal end side of the first tubular portion 210 and capable of connecting the needle hub 120.

The first tubular portion 210 and the second tubular portion 220 have a substantially cylindrical outer shape. The second tubular portion 220 disposed on the proximal end side of the first tubular portion 210 has an outer diameter larger than that of the first tubular portion 210.

When performing intradermal administration using the administration device 10, the user can grip the second tubular portion 220 positioned on the proximal end side of the syringe 200. Since the second tubular portion 220 has a larger diameter than the first tubular portion 210, the user can easily grip the second tubular portion 220, and the gripping force at the time of gripping is also increased. Therefore, when the user performs intradermal administration using the administration device 10, the needle tip 112 of the injection needle 110 protruding toward the distal end side of the administration device 10 can be firmly inserted into the administration site.

The lock mechanism 230 can be formed of, for example, a member having a screw groove on its inner surface. In the present embodiment, a connection portion 129 that can be screwed into the screw groove of the lock mechanism 230 is provided at the proximal end portion of a second member 122 of the needle hub 120. The user can detachably connect the needle hub 120 to the syringe 200 by rotating and screwing the needle hub 120 in a state where the proximal end portion of the needle hub 120 is inserted into the lock mechanism 230.

For example, the screw groove provided in the lock mechanism 230 can be formed of a female screw, and the connection portion 129 provided in the needle hub 120 can be formed of a male screw. Note, however, that the screw groove on the lock mechanism 230 side may be formed of a male screw, and the screw groove on the connection portion 129 side may be formed of a female screw. In addition, the mechanism for connecting the needle hub 120 and the syringe 200 is not particularly limited, and for example, it is also possible to adopt a mechanism in which the distal end portion of the syringe 200 is inserted inside the proximal end portion of the needle hub 120 and both are fitted. In addition, the needle hub 120 may be fixed so as not to be separable from the syringe 200 using an adhesive or the like.

As illustrated in FIG. 3, the liquid chamber 240 of the syringe 200 communicates with the lumen of the injection needle 110 in a state where the needle hub 120 and the syringe 200 are connected. As will be described later, the injection needle 110 is fixed inside the needle hub 120 by being held in a state of penetrating a holding portion 127 of an elastic member 125.

### <Needle assembly>

As illustrated in any one of FIGS. 1 to 3, the needle assembly 100 includes the injection needle 110, the needle hub 120 holding the injection needle 110, and a cap member 130 attachable to the distal end portion of the needle hub 120.

As illustrated in FIG. 3, the needle hub 120 includes a first member 121 disposed on the distal end side of the needle hub 120 and the second member 122 disposed on the proximal end side of the first member 121.

The elastic member 125 is disposed between the proximal end portion of the first member 121 and the distal end portion of the second member 122. The injection needle 110 is disposed inside the needle hub 120 in a state where a proximal end portion 113 of the injection needle 110 is aligned with the distal end of the liquid chamber 240. A fitting portion 122a into which the distal end portion of the injection needle 110 is fitted is provided on the proximal end side of the second member 122.

As illustrated in any one of FIGS. 3 to 6, the first member 121 of the needle hub 120 includes an adjustment portion 123 that adjusts the protrusion length of the needle tube 111 of the injection needle 110 by exposing a certain range from the needle tip 112 toward the proximal end side of the needle tube 111, a guide portion 124 that surrounds the adjustment portion 123 and the portion of the needle tube 111 exposed from the adjustment portion 123 and is disposed so as to form a space 124a with the adjustment portion 123 and the needle tube 111, and a flange portion 124b that extends in a planar shape at a position on the outer peripheral side of the guide portion 124.

The adjustment portion 123 is provided at a substantially central position in the plane direction of the needle hub 120. The adjustment portion 123 is formed of a hollow portion that exposes a part of the injection needle 110 on the needle tip 112 side. The adjustment portion 123 comes into contact with the surface of the administration site to define the puncture depth of the needle tube 111. Therefore, when intradermal administration is performed using the administration device 10, the needle tip 112 of the injection needle 110 punctures the administration site by a length protruding from the adjustment portion 123 to the distal end side.

The guide portion 124 is disposed in a cylindrical shape concentric with the adjustment portion 123. Therefore, as illustrated in FIG. 3, a circular space 124a is formed around the adjustment portion 123 with the adjustment portion at the center. The end surface of the guide portion 124 is positioned closer to the rear end side of the needle tube 111 than the distal end surface of the adjustment portion 123. When the needle tip 112 of the needle tube 111 punctures the administration site, the end surface of the adjustment portion 123 comes into contact with the surface of the administration site, and then the end surface of the guide portion 124 comes into contact with the surface of the administration site. Hence, the posture of the administration device 10 is stabilized and the needle tube 111 can be maintained in a posture substantially perpendicular to the administration site. Note that the shape of the guide portion 124 is not limited to the cylindrical shape, and may be formed in a polygonal cylindrical shape such as a quadrangular prism or a hexagonal prism having a cylindrical hole at the center, for example.

As illustrated in FIG. 3, the flange portion 124b extending in a planar shape is disposed at a position on the outer peripheral side of the guide portion 124. The flange portion 124b is concentric with the adjustment portion 123 and extends in the outer peripheral direction from the vicinity of the proximal end of the guide portion 124.

The elastic member 125 disposed in the vicinity of the proximal end portion 113 of the injection needle 110 enhances liquid tightness at the connection portion 129 between the liquid chamber 240 of the syringe 200 and the needle hub 120. As a result, it is possible to prevent the drug from leaking in the vicinity of the proximal end portion of the injection needle 110 when the drug is fed from the liquid chamber 240 to the lumen of the injection needle 110.

The elastic member 125 includes a deformable portion 126 that is disposed on the proximal end side and abuts on the distal end of the syringe 200, the holding portion 127 through which the injection needle 110 penetrates, and an insertion portion 128 that is disposed on the distal end side and guides the puncture direction when the injection needle 110 penetrates the holding portion 127.

Examples of the constituent material of the elastic member 125 include elastic materials such as various rubber materials like natural rubber and silicone rubber, various thermoplastic elastomers like polyurethane and styrene, and mixtures thereof.

The deformable portion 126 has a substantially cylindrical axial cross section, and is provided at an end portion of the elastic member 125 opposite to the insertion portion 128. The deformable portion 126 abuts on a distal end surface of the drug discharge portion 201 of the syringe 200. The deformable portion 126 is pushed into the distal end surface of the drug discharge portion 201 and crushed toward the insertion portion 128 at the time of assembly of the administration device 10. Since the deformable portion 126 is deformed following the distal end of the drug discharge portion 201, it is possible to reduce a dead volume caused by the difference in fitting depth of the drug discharge portion 201 to the fitting portion 122a.

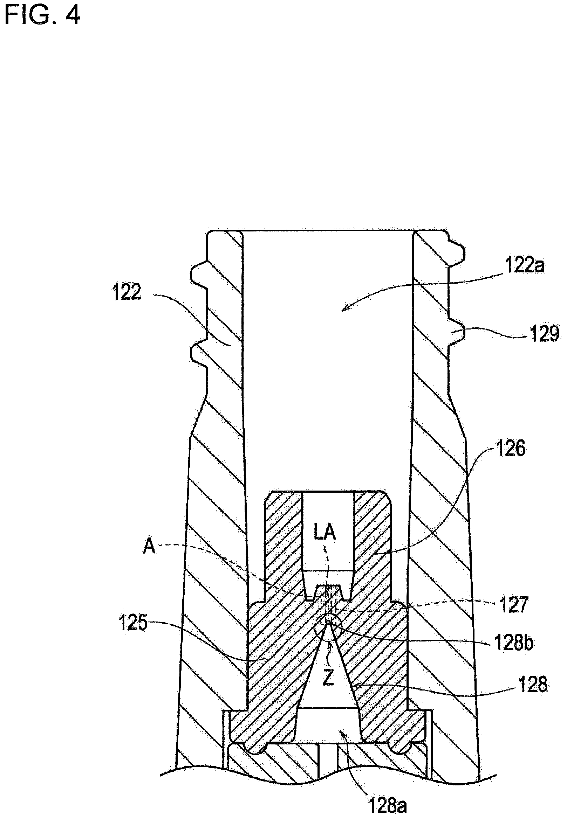

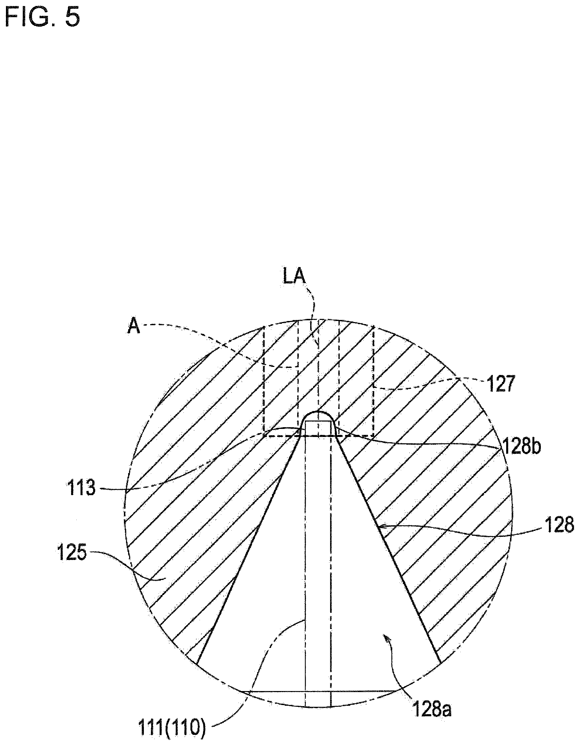

As illustrated in FIG. 4, the holding portion 127 is disposed between the internal space of the deformable portion 126 and the insertion portion 128 along the axial direction of the elastic member 125. As illustrated in FIG. 4 or 5, the holding portion 127 has a penetration region A through which the injection needle 110 penetrates, and holds the injection needle 110 in a state where a part of the needle tube 111 penetrates the penetration region A. The holding portion 127 holds the injection needle 110 in a state where the injection needle 110 penetrates from the proximal end portion 113 side through a guide portion 128b to be described later and a certain range from the proximal end portion 113 of the injection needle 110 toward the needle tip 112 is exposed.

As described above, in the needle assembly 100 according to the present embodiment, the injection needle 110 is held in a state of penetrating the holding portion 127 of the elastic member 125. The penetration hole formed in the holding portion 127 at the time of penetration is not a hole formed in advance but a hole formed by penetration, and is substantially the same as the outer diameter of the injection needle 110 that punctures the holding portion 127. Therefore, the holding portion 127 can liquid-tightly hold the injection needle 110 without generating a gap with the outer peripheral surface of the injection needle 110, and can ensure pressure resistance sufficient for administration. In addition, since the holding portion 127 holds the injection needle 110 by the penetration of the injection needle 110, it is possible to liquid-tightly hold the penetrated injection needle 110 regardless of the outer diameter size of the injection needle 110, and thus versatility is high.

The holding portion 127 only needs to include at least the penetration region A of the elastic member 125, and may include a certain range around the penetration region A. In addition, the penetration region A can be set in accordance with the outer diameter of the injection needle 110 that can be an attachment target.

The thickness of the holding portion 127 (axial length of penetration region A) is not particularly limited, but may be any thickness as long as at least the injection needle 110 can penetrate and a part of the injection needle 110 can be held in a state where the proximal end portion 113 of the injection needle 110 penetrates the holding portion 127. The guide portion 128b can suitably have a thickness of 0.5 mm or more and 1.5 mm or less, specifically, 0.9 mm when the 36G injection needle 110 is used.

The insertion portion 128 includes an opening portion 128a having a tapered axial cross section in which the inner diameter gradually decreases from the distal end side toward the proximal end side of the elastic member 125, and a guide portion 128b provided in a recessed shape from the proximal end of the opening portion 128a toward the holding portion 127.

The opening portion 128a has a tapered axial cross section in which the inner diameter gradually decreases from the distal end side toward the proximal end side of the elastic member 125. The opening portion 128a guides a rough puncture direction with respect to the holding portion 127 when the injection needle 110 penetrates the penetration region A of the holding portion 127 serving as a penetration destination. Note that the shape of the opening portion 128a is not limited in terms of a taper angle or the like as long as the injection needle 110 can easily penetrate the holding portion 127.

The guide portion 128b is a recess having a substantially semicircular axial cross section, and is provided from the proximal end of the opening portion 128a toward the holding portion 127. The guide portion 128b is fitted with the proximal end portion 113 of the injection needle 110 and guides the injection needle 110 such that the puncture direction is along a center line LA of the penetration region A. The guide portion 128b more finely guides the puncture direction of the injection needle 110 such that the axis of the injection needle 110 guided by the opening portion 128a passes through the center line LA of the penetration region A of the holding portion 127.

As described above, since the insertion portion 128 has the opening portion 128a and the guide portion 128b, when the injection needle 110 is attached to the elastic member 125, the injection needle 110 can appropriately penetrate the penetration region A of the holding portion 127 in a correct direction. Therefore, the injection needle 110 is prevented from penetrating from a direction (oblique direction) intersecting the center line LA of the penetration region A.

The opening diameter of the guide portion 128b is preferably equal to the outer diameter of the injection needle 110 to be used so that the proximal end portion 113 of the injection needle 110 accurately penetrates the penetration region A of the holding portion 127. However, the opening diameter of the guide portion 128b may be slightly larger than the outer diameter of the injection needle 110 from the viewpoint of versatility as long as the guiding to the penetration region A is not hindered.

In addition, the shape of the guide portion 128b is simple in terms of shape, and it is preferable that the axial cross section is substantially semicircular as illustrated in FIG. 5 from the viewpoint of ease of manufacturing as a mass production product.

However, the shape of the guide portion 128b is not particularly limited as long as the puncture direction of the proximal end portion 113 of the injection needle 110 is guided to the penetration region A. For example, the shape of the guide portion 128b may be a substantially U shape provided with an extending portion extending by a predetermined length from the proximal end of the opening portion 128a toward the holding portion 127, or may be other shapes.

The cap member 130 is configured to be connectable to the first member 121 of the needle hub 120. The cap member 130 is disposed so as to cover the needle tip 112 of the injection needle 110 from the distal end side of the needle hub 120 in a state of being connected to the needle hub 120. The user can prevent erroneous puncture by the needle tip 112 of the injection needle 110 by attaching the cap member 130 to the needle hub 120. In addition, the cap member 130 can always keep the used administration device 10 or needle assembly 100 in a safe state, and the user can safely dispose the used administration device 10 or needle assembly 100.

Each part of the needle hub 120 and the syringe 200 can be made of, for example, a known resin material or a known metal material. As an example, synthetic resins such as polycarbonate, polypropylene, and polyethylene, and metal materials such as stainless steel and aluminum can be used.

### <Injection needle>

The injection needle 110 includes the needle tube 111 in which a blade surface 114 is formed at the needle tip 112.

A lumen through which a drug to be intradermally administered can flow is formed inside the needle tube 111. A distal end opening communicating with the lumen is formed at the most distal end of the needle tip 112.

In the administration device 10 described above, the injection needle 110 is disposed in a state where a certain range on the proximal end side of the injection needle 110 is accommodated inside the needle hub 120 (inside first member 121 and second member 122). A part of the distal end side of the injection needle 110 is disposed so as to protrude toward the distal end side from the adjustment portion 123 of the needle hub 120.

The proximal end portion 113 of the injection needle 110 has a cylindrical shape and is held in a state of penetrating the holding portion 127 of the elastic member 125.

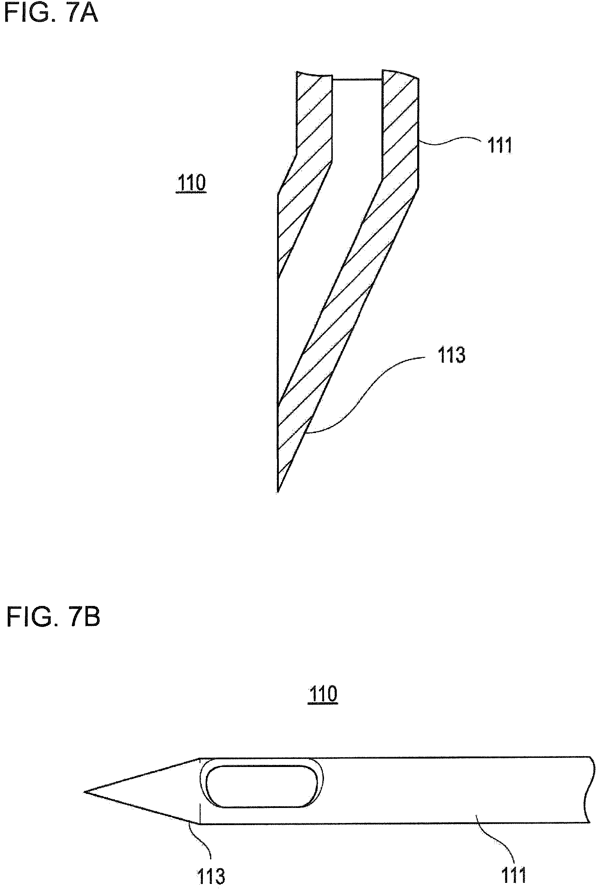

In order to prevent coring when the proximal end portion 113 penetrates the holding portion 127, it is preferable that a sharp blade portion having a configuration capable of preventing coring as illustrated in FIGS. 7A and 7B is provided. As illustrated in FIG. 7A, the proximal end portion 113 of the injection needle 110 may form a sharp blade portion, the blade surface and the jaw may be arranged side by side along the puncture direction, and the blade portion may be displaced in the radial direction with respect to the axis of the injection needle 110. With such a configuration, since the blade surface of the blade portion does not face the elastic member 125, it is possible to prevent coring during piercing. Furthermore, as illustrated in FIG. 7B, the proximal end portion 113 may have a form like a pencil point needle including a substantially pyramid-shaped blade edge and a discharge port disposed on the side of the needle tube 111 intersecting the axial direction. With such a configuration, since there is no portion that can cut the elastic member 125 on the blade edge, coring can be prevented.

The outer diameter of the injection needle 110 is not particularly limited, and can be arbitrarily selected according to the thickness of the administration site. Note, however, that since the needle assembly 100 can also be adapted to an injection needle 110 having an outer diameter size used when the biological tissue thinner than the human skin upper layer portion is the administration site as described above, the needle assembly is preferably in a range of 0.08 mm to less than 0.18 mm in consideration of rigidity and the like at the time of penetration of the holding portion 127.

The specific shape and structure of the injection needle 110 are not particularly limited. The injection needle 110 may be formed of not only a straight needle but also a tapered needle at least a part of which is tapered, or the radial cross-sectional shape of the needle tube 111 may be a polygon such as a triangle.

The injection needle 110 can be formed of, for example, a metal needle including metal as a constituent material. As the metal constituting the injection needle 110, for example, stainless steel, aluminum, an aluminum alloy, titanium, a titanium alloy, or other metals can be used.

### [Operation]

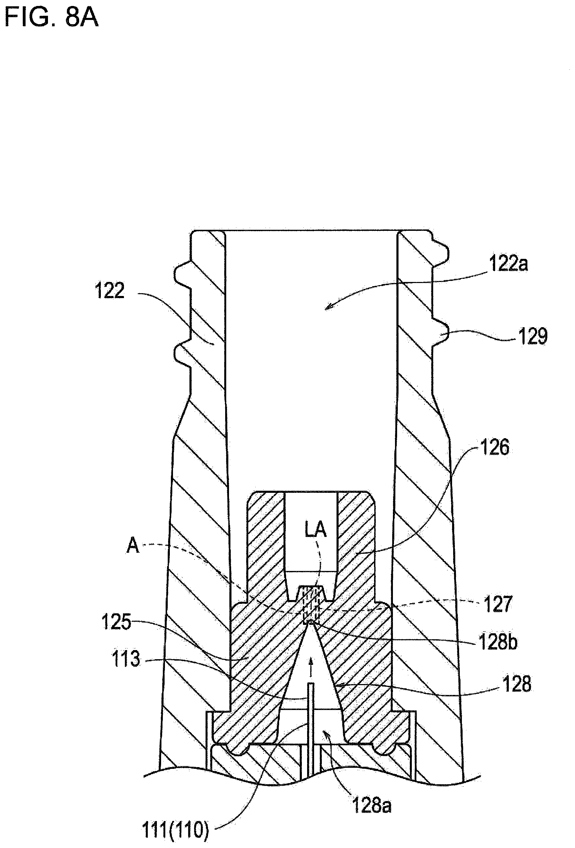

Next, operation and action at the time of attaching the injection needle 110 in the needle assembly 100 according to the present embodiment will be described with reference to FIGS. 8A to 8C. The operation illustrated in FIGS. 8A to 8C is an operation according to the attachment procedure of the injection needle 110 to the elastic member 125 in the manufacturing process.

When the injection needle 110 is attached to the needle hub 120, as illustrated in FIG. 8A, the injection needle 110 is inserted into the insertion portion 128 of the elastic member 125 from the proximal end portion 113 side. At this time, since the opening portion 128a of the insertion portion 128 is formed in a tapered shape in which the inner diameter gradually decreases from the distal end toward the proximal end, it is easy to grasp the insertion direction.

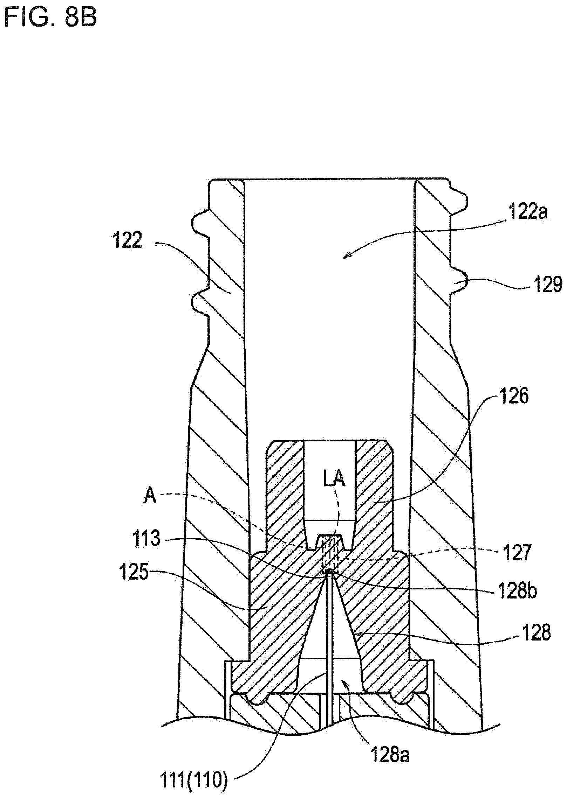

As illustrated in FIG. 8B, when the injection needle 110 continues to be inserted into the insertion portion 128 and reaches the guide portion 128b, the proximal end portion 113 is fitted into the guide portion 128b. As a result, the injection needle 110 is brought into the penetration posture in which the axial direction of the needle tube 111 and the center line LA of the penetration region A of the holding portion 127 overlap with each other. Therefore, the injection needle 110 can penetrate from a direction substantially perpendicular to the penetration region A, and is prevented from penetrating to a position deviated from the penetration region A.

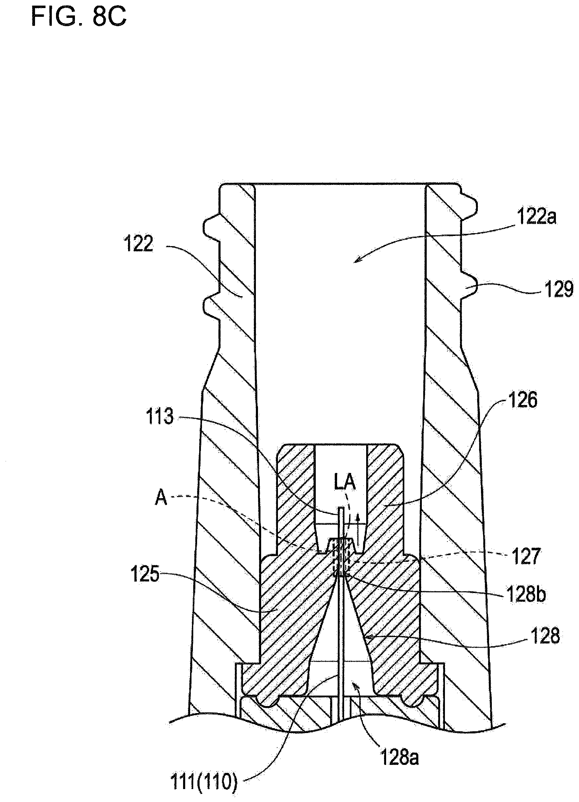

Then, the injection needle 110 penetrates the penetration region A of the holding portion 127 and is held by the holding portion 127 as illustrated in FIG. 8C. The length by which the proximal end portion 113 of the injection needle 110 protrudes from the holding portion 127 is determined according to the distance from the drug discharge portion 201 of the syringe 200 fitted into the fitting portion 122a from the proximal end side end surface of the holding portion 127.

As described above, in the needle assembly 100 according to the present embodiment, the injection needle 110 penetrates the penetration region A, and the outer peripheral surface of the injection needle 110 is liquid-tightly held substantially coinciding with the inner diameter of the through-hole drilled in the holding portion 127. Therefore, the administration device 10 in which the syringe 200 is attached to the needle assembly 100 can hold the injection needle 110 in a liquid-tight manner even if the injection needle 110 having a small outer diameter such as 36G or less is used. Hence, liquid does not leak from the elastic member 125 to the distal end side.

### [Operational effects]

As described above, the needle assembly 100 according to the present embodiment includes the injection needle 110 that includes the needle tube in which the blade surface is formed at the needle tip 112 and that is inserted into the administration site, and the needle hub 120 that holds the injection needle 110. The needle hub 120 includes the holding portion 127 that has the penetration region A through which the injection needle 110 penetrates and that holds the injection needle 110 penetrating the penetration region A, and the insertion portion 128 that is disposed on the distal end side of the holding portion 127 and that guides the proximal end portion 113 of the injection needle 110 to the penetration region A such that the puncture direction of the injection needle 110 and the center line LA of the penetration region A overlap with each other.

The penetration hole formed in the holding portion 127 by the penetration of the injection needle 110 is not a hole formed in advance, but a hole formed by the penetration, and is substantially the same as the outer diameter of the injection needle 110 that punctures the holding portion 127. Therefore, the holding portion 127 can liquid-tightly hold the injection needle 110 without generating a gap with the outer peripheral surface of the injection needle 110, and can ensure pressure resistance sufficient for administration. In addition, since the holding portion 127 holds the injection needle 110 by the penetration of the injection needle 110, the injection needle 110 can be liquid-tightly held regardless of the outer diameter of the injection needle 110.

Furthermore, in the needle assembly 100 according to the present embodiment, the insertion portion 128 includes the opening portion 128a having a tapered axial cross section in which the inner diameter gradually decreases from the distal end toward the proximal end, and the guide portion 128b that is provided in a recessed shape from the proximal end of the opening portion 128a toward the holding portion 127 and guides the puncture direction of the proximal end portion 113 of the injection needle 110 along the center line LA of the penetration region A.

With such a configuration, when the injection needle 110 is attached, the insertion direction can be easily grasped by the tapered shape of the opening portion 128a, and when the proximal end portion 113 reaches the guide portion 128b and the proximal end portion 113 is fitted into the guide portion, a penetration posture in which the axial direction of the needle tube 111 and the center line LA of the penetration region A of the holding portion 127 overlap each other is formed, and the penetration can be performed from a direction substantially perpendicular to the penetration region A.

Furthermore, in the needle assembly 100 according to the present embodiment, the guide portion 128b may have a substantially semicircular axial cross section.

With such a configuration, when the proximal end portion 113 of the injection needle 110 is fitted into the guide portion 128b, it is easy to take a puncture posture in which the axial direction of the needle tube 111 and the center line LA of the penetration region A of the holding portion 127 overlap each other. In addition, since the guide portion 128b has a simple shape, it is advantageous also from the viewpoint of ease of production as a mass production product.

Furthermore, in the needle assembly 100 according to the present embodiment, the outer diameter of the injection needle may be in the range of 0.08 mm or more and 0.18 mm or less.

Even the injection needle 110 having a very thin outer diameter of, for example, 36G or less, which can be applied to a biological tissue having a thickness smaller than the thickness of the upper layer portion of the human skin (epidermis layer and dermis layer) as the administration site, can be held in a liquid-tight manner without liquid leakage if it is attached in a state of penetrating the holding portion 127.

Furthermore, in the needle assembly 100 according to the present embodiment, the proximal end portion 113 of the injection needle 110 may have a blade portion displaced in the radial direction with respect to the axis of the injection needle. In addition, the proximal end portion 113 of the injection needle 110 may include a substantially pyramid-shaped blade edge and a discharge port disposed on the side on the proximal end side of the blade edge of the needle tube intersecting the axial direction.

With such a configuration, when the proximal end portion 113 of the injection needle 110 penetrates the holding portion 127, the holding portion 127 is not cut, and coring can be prevented.

Furthermore, in the needle assembly 100 according to the present embodiment, the administration site may be a biological tissue thinner than the thickness of the human skin upper layer portion in a human or a small non-human animal.

As described above, since the holding portion 127 holds the injection needle 110 in a liquid-tight manner in a state where the injection needle penetrates the holding portion 127, there is no need to worry about liquid leakage even if the outer diameter of the injection needle 110 is 36G or smaller, which is applied to a biological tissue thinner than the thickness of the upper layer portion of the human skin in a human or small non-human animal, for example.

In addition, the administration device 10 according to the present embodiment includes any of the needle assemblies 100 described above, and the syringe 200 that has the first tubular portion 210 disposed on the proximal end side of the needle hub 120 and the second tubular portion 220 disposed on the proximal end side of the first tubular portion 210 and having an outer diameter larger than that of the first tubular portion 210, and that is connected to the needle hub 120 of the needle assembly 100.

With such a configuration, even when the injection needle 110 having a very thin outer diameter (e.g., needle of 36G or less) used for intradermal administration to a biological tissue thinner than the upper layer portion of the human skin in a human or a small non-human animal is attached to the needle hub 120, intradermal administration can be performed without liquid leakage. In addition, since the second tubular portion 220 has a larger diameter than the first tubular portion 210, the user can easily grip the second tubular portion 220, and the gripping force at the time of gripping is also increased. Therefore, when the user performs intradermal administration using the device 10, the needle tip 112 of the injection needle 110 protruding toward the distal end side of the administration device 10 can be firmly inserted into the administration site.

### Example

Hereinafter, while the present invention will be specifically described with reference to examples, the scope of the present invention is not limited to the following examples. In the following examples, unless otherwise specified, operations were performed at room temperature (25°C).

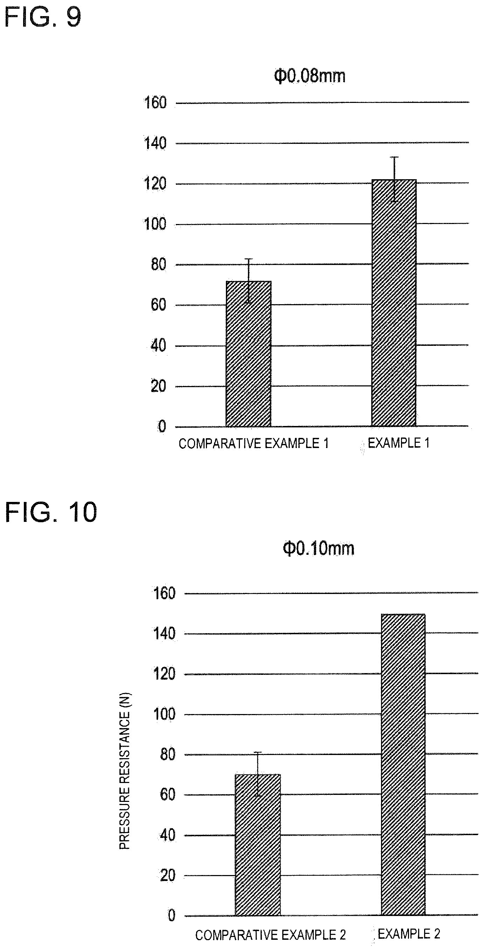

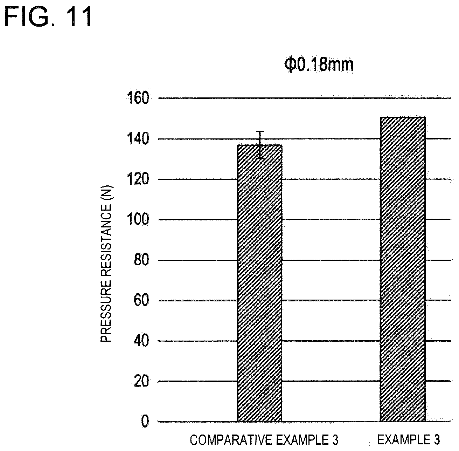

In FIGS. 9 to 11, samples using the needle assembly according to the present embodiment (Examples 1 to 3) and samples using a needle assembly of a conventional product (Comparative Examples 1 to 3) were subjected to a pressure resistance test against liquid leakage.

### <Sample specifications>

The injection needles of Example 1 and Comparative Example 1 had an outer diameter of 0.08 mm, the injection needles of Example 2 and Comparative Example 2 had an outer diameter of 0.10 mm, and the injection needles of Example 3 and Comparative Example 3 had an outer diameter of 0.18 mm. The samples of Examples 1 to 3 are needle assemblies according to the present embodiment, and hold an injection needle in a state of penetrating a holding portion of an elastic member. The samples of Comparative Examples 1 to 3 are needle assemblies of conventional products, and a mounting hole (inner diameter: 0.16 mm) for mounting an injection needle was formed in an elastic member, and the injection needle used in each sample was inserted into and held in the mounting hole. The dimension (thickness of holding part of Examples 1 to 3, total length of mounting hole of Comparative Examples 1 to 3) of the injection needle attachment portion of the elastic member used in each sample was 0.9 mm.

### <Examination conditions>

The distal end of the injection needle of each sample was blocked with an adhesive such as hot melt so as not to pass liquid, a luer lock syringe was filled with 100 µL of colored distilled water, the air in the syringe was removed, and then the syringe was connected to each sample.

The syringe equipped with each sample was set in a small desktop testing machine (EZ-L: manufactured by Shimadzu Corporation), the flange portion of the syringe was fixed, a load was applied to the plunger of the syringe at a speed of 5 mm/min in the compression direction, and the load when leakage of distilled water from the elastic member of each sample was confirmed was measured as a pressure resistance.

### <Test results>

As shown in FIGS. 9 to 11, when the pressure resistance of Examples 1 to 3 was compared with that of Comparative Examples 1 to 3, it was confirmed that the pressure resistance of the example was improved as compared with that of the comparative example in all of the test results. In particular, as shown in FIGS. 9 and 10, in Comparative Example 1 (injection needle with outer diameter of 0.08 mm) and Comparative Example 2 (injection needle with outer diameter of 0.10 mm), since the injection needle is inserted into the mounting hole having an inner diameter of 0.16 mm close to the manufacturing limit lower limit value, there is a gap between the injection needle and the mounting hole. Therefore, it is presumed that liquid leaked at a low pressure resistance when a load was applied. On the other hand, as shown in FIGS. 9 and 10, in Examples 1 and 2, since the injection needle is held in a state of penetrating the holding portion, the injection needle can be held in a liquid-tight manner regardless of the outer diameter of the injection needle. Therefore, it is presumed that a higher pressure resistance than that of the comparative example was obtained. From the above test results, it was shown that since the needle assembly according to the present invention has a configuration in which the injection needle is held by penetrating the holding portion, pressure resistance can be significantly improved as compared with a configuration in which the injection needle is mounted in a mounting hole formed in advance as in a conventional product.

The present application is based on Japanese Patent Application No. 2022-004167 filed on January 14, 2022, the disclosure content of which is incorporated herein by reference in its entirety.

### Reference Signs List

- 10: Administration device
- 100: Needle assembly
- 110: Injection needle
- 111: Needle tube
- 112: Needle tip
- 113: Proximal end portion
- 120: Needle hub
- 121: First member
- 122: Second member
- 125: Elastic member
- 127: Holding portion
- 128: Insertion portion
- 128a: Opening portion
- 128b: Guide portion
- 130: Cap member
- 200: Syringe
- 210: First tubular portion
- 220: Second tubular portion
- 300: Plunger
- A: Penetration region of the holding portion
- LA: Center line of the penetration region

## Claims

1. A needle assembly comprising:
an injection needle that includes a needle tube in which a blade surface is formed at a needle tip and that is inserted into an administration site; and
a needle hub that holds the injection needle, wherein
the needle hub includes
a holding portion that has a penetration region through which the injection needle penetrates and that holds the injection needle penetrating the penetration region, and
an insertion portion that is disposed on a distal end side of the holding portion and that guides a proximal end portion of the injection needle to the penetration region such that a puncture direction of the injection needle and a center line of the penetration region overlap with each other.

2. The needle assembly according to claim 1, wherein
the insertion portion includes an opening portion having a tapered axial cross section in which an inner diameter gradually decreases from the distal end toward the proximal end, and
a guide portion that is provided in a recessed shape from the proximal end of the opening portion toward the holding portion and guides the puncture direction of the proximal end portion of the injection needle along the center line of the penetration region.

3. The needle assembly according to claim 2, wherein the guide portion has a substantially semicircular axial cross section.

4. The needle assembly according to any one of claims 1 to 3, wherein an outer diameter of the injection needle is in a range of 0.08 mm or more and 0.18 mm or less.

5. The needle assembly according to any one of claims 1 to 4, wherein the proximal end portion of the injection needle has a blade portion displaced in a radial direction with respect to an axis of the injection needle.

6. The needle assembly according to any one of claims 1 to 4, wherein the proximal end portion of the injection needle includes a substantially pyramid-shaped blade edge and a discharge port disposed on a side on the proximal end side of the blade edge of the needle tube intersecting an axial direction.

7. The needle assembly according to any one of claims 1 to 6, wherein the administration site is a biological tissue thinner than an upper layer portion of a human skin in a human or a small non-human animal.

8. An administration device comprising:
the needle assembly according to any one of claims 1 to 7; and
a syringe that has a first tubular portion disposed on the proximal end side of the needle hub and a second tubular portion disposed on the proximal end side of the first tubular portion and having an outer diameter larger than an outer diameter of the first tubular portion, and that is connected to the needle hub of the needle assembly.
